# FASCICULE DE BREVET EUROPEEN

(11) **EP 1 228 075 B1**
(45) Date de publication et mention de la délivrance du brevet: **18.06.2003**
(21) Numéro de dépôt: 00966244.6
(22) Date de dépôt: 29.09.2000
(51) Int. Cl.: C07F 7/08, C07F 7/21, C08G 77/38, A61K 7/42, A61K 31/695

(54) **COMPOSES SILICIES DERIVES DE L'ACIDE ASCORBIQUE**
SILIZIUM-ENTHALTENDE ASCORBINSÄUREDERIVATE
SILICON COMPOUNDS DERIVED FROM ASCORBIC ACID

(30) Priorité: 26.10.1999 FR 9913355
(43) Date de publication de la demande: 07.08.2002
(73) Titulaire: L'OREAL, 75008 Paris (FR)
(72) Inventeur: XU, Jinzhu, F-75014 Paris (FR); RICHARD, Hervé, F-93420 Villepinte (FR)
(74) Mandataire: Dodin, Catherine
(86) Numéro de dépôt international: FR0002713
(87) Numéro de publication internationale: WO01030784

(56) Documents cités:
- EP-A- 0 766 129
- WO-A-92/17184
- US-A- 5 843 411

## Description

La présente invention concerne de nouveaux dérivés siliciés d'acide ascorbique, liposolubles et stables. L'invention concerne également des compositions notamment cosmétiques ou pharmaceutiques comprenant ces nouveaux dérivés, ainsi que leur utilisation.

On connaît un certain nombre de dérivés du silicium. Ainsi, la demande de brevet FR2645863 décrit des complexes moléculaires formés d'un composé appartenant à la famille des silanols et d'un dérivé alcalin ou ammonium d'un acide organique ou minéral. On obtient ainsi des combinaisons moléculaires qui présentent l'avantage d'être solubles dans l'eau. Ce document cite notamment le complexe moléculaire formé d'un monométhylsilane triol et d'ascorbate de potassium. Toutefois, ainsi qu'il est spécifié dans ce document, la stabilité de ces complexes n'est pas très bonne, notamment lorsqu'ils sont en solution concentrée. En particulier, ils ont tendance à se polymériser ce qui provoque leur insolubilisation partielle.
On connaît également, par la demande de brevet WO96/10575, un dérivé bis(diméthylsilyl)-2,3-5,6-ascorbate ou ascorbosilyl; dans ce cas, il s'agit d'un précurseur de fonctions silanols réactives, donc non stable également.

La présente invention a pour but de proposer de nouveaux composés siliciés dérivés de l'acide ascorbique, lesdits composés étant liposolubles et présentant une bonne stabilité tant à l'état sec qu'en solution diluée ou concentrée.

L'invention a donc pour objet de nouveaux composés siliciés dérivés de l'acide ascorbique tels que définis ci-après.

Un autre objet de l'invention est un procédé de préparation des composés de formules (2) à (4) ci-après définies, par hydrosilylation du dérivé siloxanique ou silanique correspondant représenté par l'une des formules (5) à (7) ci-après définies,
sur un dérivé organique d'acide ascorbique choisi parmi les composés de formule (I') ci-après définie; suivie d'une déprotection du composé obtenu, par exemple par hydrolyse acide des isopropylidènes et/ou débenzylation par hydrogénation catalytique.

Un autre objet de l'invention est un procédé de préparation des dérivés silaniques de formule (2) ci-après définie, par réaction d'un dérivé de formule (I"') ci-après définie, avec un dérivé silanique de formule (8) suivante : 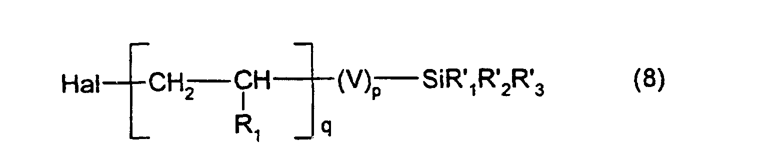 dans laquelle Hal représente un halogène et plus particulièrement le chlore ou l'iode et les radicaux R₁, R'₁, R'₂, R'₃, V, p et q ont les mêmes significations que ci-dessus.

Encore un autre objet de l'invention est une composition notamment cosmétique ou pharmaceutique, comprenant un milieu cosmétiquement ou pharmaceutiquement acceptable et au moins un composé silicié dérivé de l'acide ascorbique tel que défini ci-dessus.

Un autre objet de l'invention est l'utilisation d'au moins un tel composé silicié dérivé de l'acide ascorbique en tant qu'agent antioxydant et/ou agent anti-radicaux libres, notamment dans une composition cosmétique ou pharmaceutique.

En particulier, cette utilisation peut être cosmétique pour, ou dans une composition cosmétique destinée à, traiter le stress oxydant et/ou traiter les effets de l'exposition au soleil et/ou prévenir le vieillissement notamment de la peau, des cheveux, des cils, des sourcils et/ou des ongles.

Cette utilisation peut également être pour la préparation d'une composition pharmaceutique destinée à traiter le stress oxydant et/ou traiter les effets de l'exposition aux rayonnements ionisants ou solaires, et/ou prévenir le vieillissement notamment de la peau, des cheveux, des cils, des sourcils et/ou des ongles, et/ou traiter les effets de l'utilisation de certains médicaments générateurs de radicaux libres.

Ainsi, la demanderesse a trouvé qu'en greffant de manière covalente sur une chaîne siliconée, un ou plusieurs dérivés d'acide ascorbique, on obtenait des composés nouveaux qui présentaient de très bonnes propriétés de solubilité dans les solvants organiques usuels, notamment dans les corps gras tels que les huiles, ainsi qu'une grande stabilité dans ces milieux ou en tant que tels.
Par ailleurs, on a constaté que ces composés avaient d'excellentes propriétés cosmétiques.

Les composés selon l'invention sont donc caractérisés par le fait
- soit qu'ils sont constitués d'une chaîne siliconée comportant au moins une unité de formule (1) : 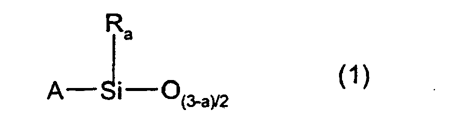
- soit qu'ils sont des silanes répondant à la formule (2) suivante :

   A-SiR^{'}₁R^{'}₂R^{'}₃ (2)
dans lesquelles :
- R désigne un radical hydrocarboné, linéaire, cyclique ou ramifié, saturé ou insaturé en C₁-C₃₀, éventuellement partiellement ou totalement halogéné, ou un groupe triméthylsilyloxy de formule -O-SiMe₃;
- a est égal à 1 ou 2,
- R'₁, R'₂, R'₃ , identiques ou différents, sont choisis parmi les radicaux alkyles linéaires ou ramifiés en C₁-C₈, les radicaux alcényles linéaires ou ramifiés en C₁-C₈, ou un groupe triméthylsilyloxy;
- A est un radical de formule (I) suivante : 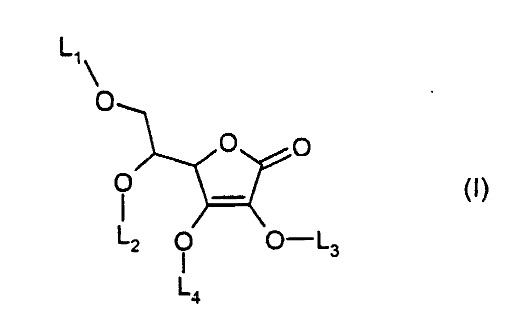
dans laquelle L₁, L₂, L₃ et L₄ représentent l'hydrogène ou un radical divalent de formule (a) ou (a') permettant l'accrochage du radical A sur la chaîne siliciée, sous réserve qu'au moins un des radicaux L₁, L₂, L₃ et L₄, de préférence un seul desdits radicaux L₁, L₂, L₃ et L₄, représente ledit radical divalent de formule (a) ou (a') suivante : 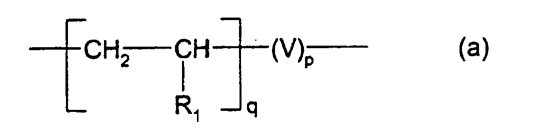 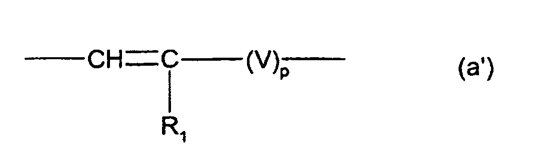 dans lesquelles :
- V est un radical hydrocarboné divalent en C₁-C₆ linéaire ou ramifié, saturé ou insaturé, éventuellement substitué par un radical hydroxyle ou un radical alcoxy en C₂-C₈, linéaire ou ramifié, saturé ou insaturé;
- R₁ représente un atome d'hydrogène, un radical hydroxyle ou un radical hydrocarboné en C₁-C₈, linéaire ou ramifié, saturé ou insaturé;
- p est 0 ou 1, q est 0 ou 1, étant donné que p + q est différent de 0.

Les composés selon l'invention constitués d'une chaîne siliconée comportant au moins une unité de formule (1) ci-dessus comprennent donc, notamment, au moins une unité de formule (1a): 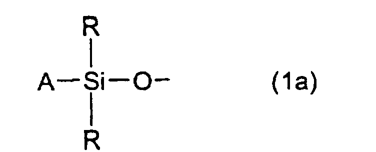 et/ou au moins une unité de formule (1b) :

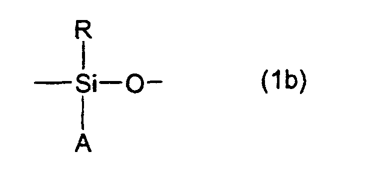

De préférence, ils peuvent comprendre en outre au moins une autre unité, par exemple de type (di-)alkylsiloxane de formule (1c) : 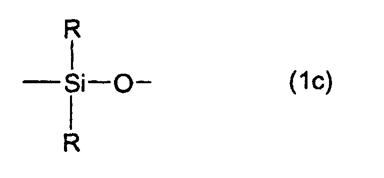 Ainsi, ils peuvent notamment être représentés par l'une des formules (3) ou (4) suivantes : 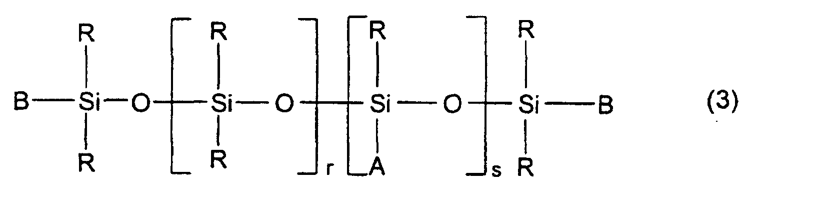 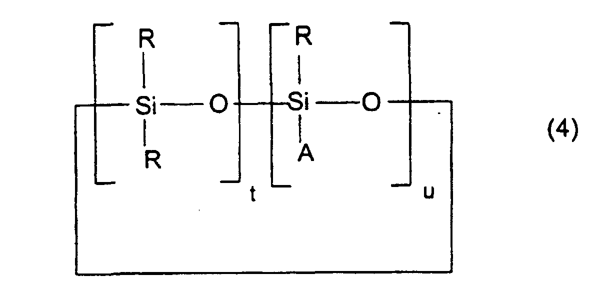 dans lesquelles :
- les radicaux B, identiques ou différents, sont choisis parmi les radicaux R et A,
- r est un nombre entier compris entre 0 et 50 inclus, de préférence entre 0 et 5,
- s est un nombre entier compris entre 0 et 20 inclus, de préférence choisi parmi 0, 1 ou 2, sous réserve que si s=0, alors au moins l'un des deux radicaux B, représente A,
- u est un nombre entier compris entre 1 et 6 inclus, de préférence choisi parmi 1 ou 2,
- t est un nombre entier entre 0 et 9 inclus, de préférence choisi parmi 2, 3 ou 4,
- étant donné que t + u est compris entre 3 et 10 inclus, de préférence vaut 3, 4, 5 ou 6.

De préférence, les radicaux R, identiques ou différents, sont choisis parmi les radicaux hydrocarbonés en C₁-C₁₈, linéaires, cycliques ou ramifiés, saturés ou insaturés, et les radicaux hydrocarbonés en C₁-C₈, linéaires ou ramifiés, saturés ou insaturés, partiellement halogénés, notamment fluorés.
Encore plus préférentiellement, on choisit les radicaux R, identiques ou différents, parmi les radicaux alkyles en C₁-C₁₀, linéaires ou ramifiés; le radical phényle; les radicaux alkyles fluorés en C₁-C₈, linéaires ou ramifiés.
On peut particulièrement citer les radicaux méthyle et 3,3,3-trifluoropropyle. Plus particulièrement, au moins 80% en nombre des radicaux R sont des radicaux méthyle.

De préférence, les radicaux R'₁, R'₂, R'₃, identiques ou différents, sont choisis parmi les radicaux alkyles linéaires ou ramifiés en C₁-C₆ , notamment méthyle ou éthyle, et le groupe triméthylsilyloxy.

D'une manière générale, on préfèrera plus particulièrement les dérivés siliciés répondant à l'une des formules (1), (2), (3) ou (4), et présentant au moins l'une des caractéristiques suivantes :
- R est CH₃;
- B est CH₃;
- R'₁, R'₂, R'₃ représentent CH₃ ou le groupe triméthylsilyloxy;
- R₁ est l'hydrogène ou CH₃;
- V est -CH₂- ou -CH₂-CH(OH)CH₂-;
- p est 1,
- r est compris entre 0 et 5 inclus,
- s est compris entre 1 et 2 inclus,
- t + u est compris entre 3 et 6.

De préférence, les radicaux divalents correspondant aux formules (a) ou (a') sont choisis parmi les radicaux hydrocarbonés, linéaires ou ramifiés, saturés ou insaturés, éventuellement hydroxylés, divalents en C₁-C₆ tels que les radicaux méthylène (-CH₂-), éthylène (-CH₂-CH₂-), propylène (-CH₂-CH₂-CH₂-), n-butylène (-CH₂-CH₂-CH₂-CH₂-), iso-butylène (-CH₂-CH(CH₃)-CH₂-), les radicaux -CH=CH-CH₂- ,
-CH=C(CH₃)-CH₂-, -CH=CH-CH(CH₃)- et -CH₂-CH(OH)-CH₂-.

Parmi les dérivés siliciés préférentiels répondant à la formule (1), et plus particulièrement à la formule (3), on peut citer les composés suivants :
- la 5-(1,2-dihydroxy-éthyl)-3-hydroxy-4-(3-[1,3,3,3-tetraméthyl-1-[(triméthylsilyl) oxy]disiloxanyl]propyloxy)-5H-furan-2-one,
- la 5-(1,2-dihydroxy-éthyl)-4-hydroxy-3-(3-[1,3,3,3-tetraméthyl-1-[(triméthylsilyl) oxy]disiloxanyl]propyloxy)-5H-furan-2-one,
- la 5-(1,2-dihydroxy-éthyl)-3-hydroxy-4-[2-méthyl-3-[1,3,3,3-tetraméthyl-1-[(triméthylsilyl)oxy]disiloxanyl]propyloxy]-5H-furan-2-one,
- la 5-(1,2-dihydroxy-éthyl)-4-hydroxy-3-[2-méthyl-3-[1,3,3,3-tetraméthyl-1-[(triméthylsilyl)oxy]disiloxanyl]propyloxy]-5H-furan-2-one,
- la 5-(1,2-dihydroxy-éthyl)-4-[3-[3-[2-méthyl-3-[1,3,3,3-tetraméthyl-1-[(triméthylsilyl)oxy]disiloxanyl]propyloxy]-2-hydroxy-propyloxy]-3-hydroxy-5H-furan-2-one et
- la 5-(1,2-dihydroxy-éthyl)-3-[3-[3-[2-méthyl-3-[1,3,3,3-tetraméthyl-1-[(triméthylsilyl)oxy]disiloxanyl]propyloxy]-2-hydroxy-propyloxy]-4-hydroxy-5H-furan-2-one.

Parmi les dérivés siliciés préférentiels répondant à la formule (2), on peut citer les composés suivants :
- la 5-(1,2-dihydroxy-éthyl)-3-hydroxy-4-triméthylsilanylméthoxy-5H-furan-2-one,
- la 5-(1,2-dihydroxy-éthyl)-4-hydroxy-3-(3-triméthylsilanylméthoxy)-5H-furan-2-one,
- la 5-(1,2-dihydroxy-éthyl)-3-hydroxy-4-(3-triméthylsilanylpropyloxy)-5H-furan-2-one, et
- la 5-(1,2-dihydroxy-éthyl)-4-hydroxy-3-(3-triméthylsilanylpropyloxy)-5H-furan-2-one

Pour préparer les dérivés de formules (1) à (4), on peut procéder classiquement en mettant en oeuvre une réaction d'hydrosilylation à partir du dérivé siloxanique ou silanique correspondant dans lequel, par exemple, tous les radicaux A sont des atomes d'hydrogène. Ce dérivé siloxanique ou silanique est dénommé dans la suite de la présente description "dérivé à SiH".

Les groupes SiH peuvent être présents dans la chaîne et/ou aux extrémités de la chaîne siliciée. Ces dérivés à SiH sont des produits bien connus dans l'industrie des silicones et sont généralement disponibles dans le commerce. Ils sont par exemple décrits dans les brevets US-A-3220972, US-A-3697473 et US-A-4340709.

Les dérivés à SiH permettant la préparation des composés de formule (2) à (4) peuvent être représentés par les formules (5) à (7) suivantes :

H-SiR^{'}₁R^{'}₂R^{'}₃ (5)

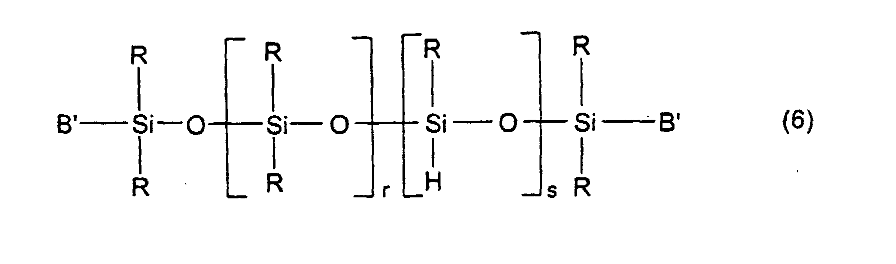

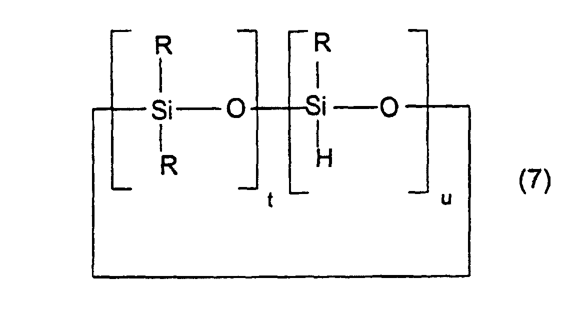

dans lesquelles :
- R'₁, R'₂, R'₃, R, r, s, t et u ont la signification donnée ci-dessus,
- B', identiques ou différents, sont choisis parmi les radicaux R et un atome d'hydrogène, sous réserve que si s=0, alors au moins l'un des deux radicaux B' représente H.

Afin de préparer les composés selon l'invention de formule (2) à (4) ci-dessus, on peut procéder de la manière suivante.

On effectue une réaction d'hydrosilylation du dérivé à SiH de formule (5), (6) ou (7), de préférence en présence d'une quantité catalytiquement efficace d'un catalyseur au platine, sur un dérivé organique d'acide ascorbique choisi parmi les composés de formule (I') suivante : 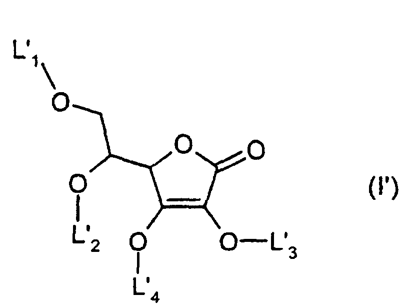 dans laquelle L'₁, L'₂, L'₃ et L'₄ représentent des groupements benzyloxy ou répondent à l'une des deux formules (b) et (b') suivantes : 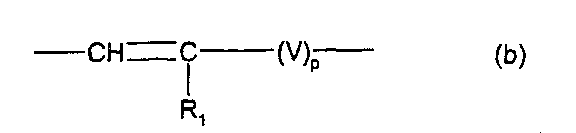 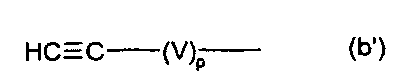 dans lesquelles R₁, V et p ont les mêmes significations que ci-dessus,
les radicaux L'₁ et L'₂ pouvant en outre former ensemble avec le reste d'acide ascorbique un cycle méthylène dioxy substitué par au moins un groupement alkyle en C₁₋₆ ou un groupement phényle, notamment par un groupement méthyle, éthyle
ou phényle, voire par deux groupements alkyles ou phényles; sous réserve qu'au moins un des radicaux L'₁, L'₂, L'₃ et L'₄ représente le radical (b) ou (b').

La réaction d'hydrosilylation peut donc s'effectuer selon l'une des deux réactions suivantes : 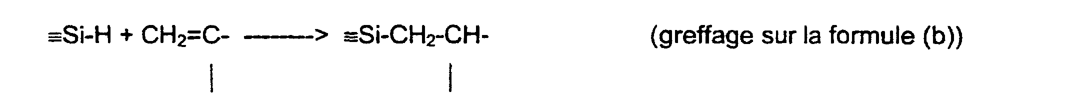 ou

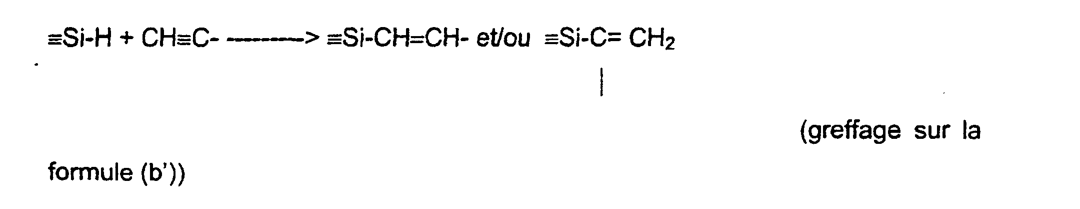

Ces dérivés (I') peuvent notamment être obtenus par condensation, de manière classique, d'un halogénure d'alcène ou d'alcényle sur un dérivé de formule (I") : 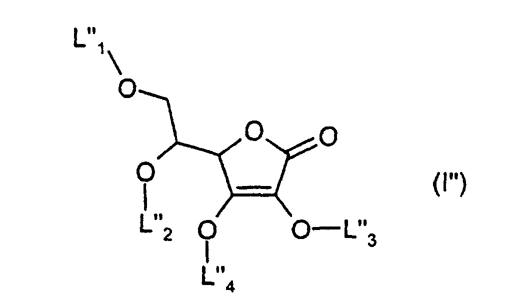 dans laquelle L"₁, L"₂, L"₃ et L"₄ représentent des groupements benzyloxy ou un atome d'hydrogène, sous réserve qu'au moins un des radicaux L"₁, L"₂, L"₃ et L"₄ représente l'hydrogène.
Ces dérivés de formule (I") peuvent être préparés selon les modes opératoires usuels bien connus de l'homme du métier; certains sont notamment décrits dans la demande de brevet EP411184.

Après l'hydrosilylation du dérivé à SiH de formules (5), (6) ou (7) sur le dérivé d'acide ascorbique de formule (I'), on peut effectuer une déprotection du composé obtenu, selon les méthodes classiques de déprotection connues dans la littérature, par exemple par hydrolyse acide des isopropylidènes et/ou débenzylation par hydrogénation catalytique.
On obtient alors les composés de formules (2) à (4) ci-dessus selon l'invention.

Par ailleurs, il est possible de préparer les dérivés silaniques de formule (2) selon un autre procédé de synthèse qui consiste à faire réagir :
- un dérivé de formule (I"') : 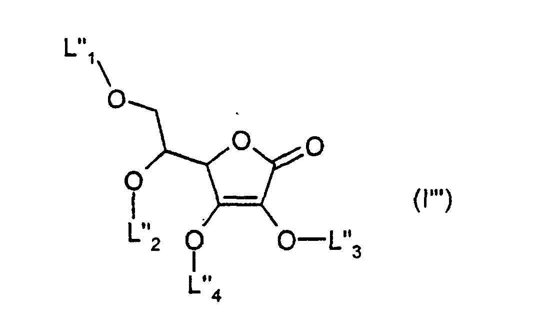
dans laquelle L"₁, L"₂, L"₃ et L"₄ représentent des groupements benzyloxy, un atome d'hydrogène,
les radicaux L"₁ et L''₂ pouvant en outre former ensemble avec le reste d'acide ascorbique un cycle méthylène dioxy substitué par au moins un groupement alkyle en C₁₋₆ ou un groupement phényle, notamment par un groupement méthyle, éthyle ou phényle, voire par deux groupements alkyles ou phényles;
sous réserve qu'au moins un des radicaux L"₁, L"₂, L''₃ et L"₄ représente l'hydrogène,
- avec un dérivé silanique de formule (8) suivante : 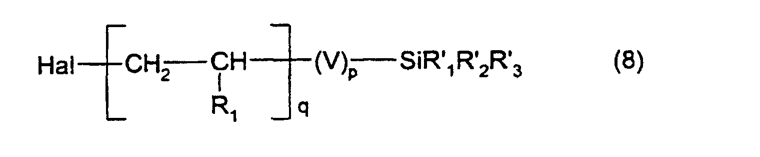
dans laquelle Hal représente un halogène et plus particulièrement le chlore ou l'iode et les radicaux R₁, R'₁, R'₂, R'₃, V, p et q ont les mêmes significations que ci-dessus.

Les composés selon l'invention sont généralement présents dans les compositions de l'invention dans des proportions comprises entre 0,1% et 10% en poids, de préférence entre 0,5% et 5% en poids, par rapport au poids total de la composition.

Ces compositions, qui peuvent notamment être des compositions cosmétiques ou pharmaceutiques, comprennent donc par ailleurs un milieu cosmétiquement ou pharmaceutiquement acceptables, c'est-à-dire un milieu compatible avec toutes les matières kératiniques telles que la peau du corps ou du visage, les muqueuses, les semi-muqueuses, le cuir chevelu, ainsi que les phanères telles que les ongles, les cheveux, les cils et les sourcils.

Les compositions selon l'invention peut se présenter sous toute forme galénique cosmétiquement ou pharmaceutiquement acceptable, telle que sous forme d'une lotion, suspension, dispersion, solution en milieu solvant ou hydroalcoolique, éventuellement multiphasée, éventuellement épaissie voire gélifiée; sous forme d'un gel, d'une mousse, d'un spray, d'une émulsion huile-dans-eau, eau-dans-huile ou multiple; sous forme de poudre libre, compacte ou coulée; sous forme d'un solide ou d'une pâte anhydre.
L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.

Ainsi, la composition peut comprendre au moins un ingrédient choisi parmi les adjuvants habituellement utilisés dans le domaine considéré, tels que des corps gras, des solvants organiques, de l'eau, des silicones, des épaississants, des adoucissants, des filtres solaires, des agents anti-mousses, des agents hydratants, des parfums, des conservateurs, des tensioactifs, des charges, des séquestrants, des polymères anioniques, cationiques, non ioniques et/ou amphotères, des propulseurs, des agents alcalinisants ou acidifiants, des colorants, des pigments ou nanopigments, des actifs cosmétiques.

Parmi les corps gras, on peut citer les huiles et /ou les cires, notamment d'origine animale, végétale, minérale ou de synthèse; les acides gras en C₈-C₃₂; les esters d'acides gras en C₈-C₃₂; les alcools gras en C₈-C₃₂. On peut plus particulièrement citer la vaseline, la paraffine, la lanoline, la lanoline hydrogénée, la lanoline acétylée, l'huile de palme hydrogénée, l'huile de ricin hydrogénée, l'huile de vaseline, l'huile de paraffine, l'huile de Purcellin, les huiles de silicones, volatiles ou non, les isoparaffines.
Parmi les solvants organiques, on peut citer les alcools et polyols inférieurs en C₁-C₆, tels que l'éthanol, l'isopropanol, le propylèneglycol, la glycérine et le sorbitol.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

On a constaté que les composés de l'invention présentent une excellente liposolubilité, notamment dans les corps gras tels que le Miglyol, les alcools (éthanol notamment), les glycols (propylène glycol notamment) et les huiles siliconées (PDMS notamment). Par ailleurs, ils se répartissent uniformément dans les supports cosmétiques classiques contenant au moins une phase grasse ou un solvant organique cosmétiquement acceptable.
Par ailleurs, on a constaté que les composés selon l'invention pouvaient présenter de bonnes propriétés antioxydantes et/ou anti radicaux libres. Ils trouvent donc une application tout particulière dans les compositions cosmétiques ou pharmaceutiques destinées à traiter le stress oxydant et/ou traiter les effets de l'exposition au soleil et/ou prévenir le vieillissement notamment de la peau, des cheveux, des cils, des sourcils et/ou des ongles.

Les compositions les comprenant trouvent donc une application toute particulière comme composition à appliquer sur la peau du corps et/ou du visage, et/ou sur les cheveux, cils, sourcils et/ou ongles.
Notamment, ces compositions peuvent se présenter être utilisées :
- comme composition protectrice de l'épiderme humain ou des cheveux contre les UV (compositions antisolaires);
- comme composition cosmétique pour la protection ou de traitement ou soin des cheveux, notamment sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux;
- comme composition de soin de la peau du corps et/ou du visage, telle que crème de traitement de l'épiderme, crème de jour, de nuit, crème anti-rides, crème hydratante, crème pour les mains ou les pieds;
- comme composition de maquillage des cils, des sourcils, des cheveux, du corps
ou du visage, telle que fond de teint, rouge à lèvres, fards à paupières, fards à joues, ligneur encore appelé "eye-liner", mascara, gel colorant, vernis à ongles.

L'invention est illustrée plus en détails dans les exemples qui suivent.

### Exemple 1: Préparation du 5-(1,2-dihydroxy-éthyl)-3-hydroxy-4-triméthylsilanylméthoxy-5H-furan-2-one

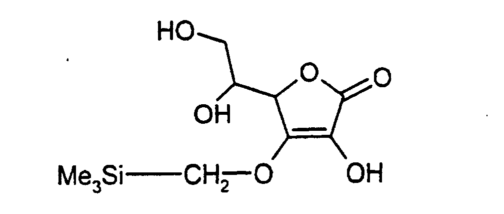

### a) 1^{ère} étape : préparation du 5-(2,2-diméthyl-[1,3]dioxolan-4-yl)-3-hydroxy-4-triméthylsilanylméthoxy-5H-furan-2-one

On ajoute, sous azote, 19,26 g (0,09 mole) d'iodométhyl-triméthylsilane à un mélange de 12,96 g (0,06 mole) de 5-(2,2-diméthyl-[1,3]dioxolan-4-yl)-3,4-dihydroxy-5H-furan-2-one (préparé selon M.E. Jung et al. J. Am. Chem. Soc., 1980, 102, 6304) et de 7,56 g (0,09 mole) de bicarbonate de sodium dans 75 ml de diméthylsulfoxyde (DMSO).
On chauffe le tout à 55°C pendant 19 heures. On refroidit et on verse dans 150 ml d'eau. On agite 1 heure à température ambiante (25°C). On filtre le précipité. On le rince à l'eau et on le sèche sous vide.
On obtient 11,1 g (rendement 61%) de produit recherché sous la forme d'un solide blanc.
Spectre H¹ RMN (CDCl₃, 200MHz) : 4,40 (d, J=4Hz, 1H) ; 4,15 (m, 2H) ; 4,07 (m, 1H) ; 3,89 (m, 2H) ; 1,28 (s, 3H) ; 1,25 (s, 3H) ; 0,00 (s, 9H).

### b) 2^{ème} étape : préparation du 5-(1,2-dihydroxy-éthyl)-3-hydroxy-4-trimethylsilanylméthoxy-5H-furan-2-one

On chauffe à 50°C le dérivé précédent (6,4 g, 0,021 mole) dans un mélange de 80 ml de méthanol et de 40 ml de HCI 2N aqueux, pendant 2 heures. On évapore le mélange sous vide à 40°C pour éliminer la plupart du méthanol. On verse le résidu dans 200 ml d'eau. On neutralise le mélange avec du bicarbonate de sodium. On extrait à l'acétate d'éthyle. On lave la phase organique à l'eau salée et la sèche sur Na₂SO₄. On évapore le solvant.
On obtient une huile incolore qui est purifiée sur silice (éluant : cyclohexane/acétate d'éthyle 50/50). On obtient 4,0 g (rendement 72%) du produit recherché sous la forme d'une huile incolore.
Spectre H¹ RMN (CDCl₃, 200MHz) : 4,54 (d, J=2Hz, 1H) ; 4,22 (m, 2H) ; 3,80 (m, 3H) ; 0,01 (s, 9H).

### Exemple 2 : Préparation du 5-(1,2-dihydroxy-éthyl)-4-hydroxy-3-(3-triméthylsilanylméthoxy)-5H-furan-2-one

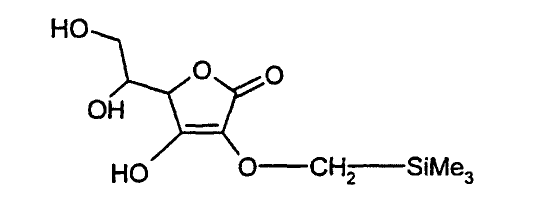

### a) 1^{ère} étape : préparation du 4-benzyloxy-5-(2,2-diméthyl-[1,3]dioxolan-4-yl)-3-hydroxy-5H-furan-2-one

A un mélange de 5-(2,2-diméthyl-[1,3]dioxolan-4-yl)-3,4-dihydroxy-5H-furan-2-one (21,62 g, 0,1 mole) et de bicarbonate de sodium (12,6 g, 0,15 mole) dans 125 ml de DMSO, sous azote, on ajoute du bromure de benzyle (13,0 ml, 0,11 mole). On chauffe le mélange à 55°C pendant 16 heures. On le refroidit et on le verse dans 250 ml d'eau. On extrait à l'acétate d'éthyle. On lave la phase organique à l'eau salée et la sèche sur Na₂SO₄. On évapore le solvant.
On obtient une huile brune qui est purifiée sur silice (éluant : cyclohexane/acétate d'éthyle 75/25) pour donner 13,8 g (rendement 45%) du produit recherché sous la forme d'une huile légèrement jaune.
Spectre H¹ RMN (CDCl₃, 200MHz) : 7,56 (m, 5H) ; 5,77 (s, 2H) ; 4,77 (d, J=4Hz, 1H) ; 4,46 (m, 1H) ; 4,20 (m, 2H) ; 1,55 (s, 3H) ; 1,48 (s, 3H).

### b) 2^{ème} étape : préparation du 4-benzyloxy-5-(2,2-diméthyl-[1,3]dioxolan-4-yl)-3-triméthylsilanylméthoxy-5H-furan-2-one

A un mélange du produit précédent (8,0 g, 0,026 mole) et de carbonate de potassium (4,32 g, 0,031 mole), sous azote, on ajoute de l'iodométhyltriméthylsilane (6,15 g, 0,029 mole). On agite le mélange à température ambiante pendant 48 heures. On le verse dans 200 ml d'eau. On extrait à l'acétate d'éthyle. On lave la phase organique à l'eau salée et la sèche sur Na₂SO₄.
Après évaporation du solvant, on obtient une huile orangée (10,5 g) qui est utilisée directement dans l'étape suivante.
Spectre H¹ RMN (CDCl₃, 200MHz) : 7,27 (m, 5H) ; 5,34 (s, 2H) ; 4,40 (d, J=3Hz, 1H) ; 4,15 (m, 1H) ; 3,99 (m, 2H) ; 3,77 (m, 2H) ; 1,28 (s, 3H) ; 1,24 (s, 3H) ; 0,00 (s, 9H).

### c) 3^{ème} étape : préparation du 4-benzyloxy-5-(1,2-dihydroxy-éthyl)-3-trimethylsilanylmethoxy-5H-furan-2-one

On dissout le produit précédent (10,5 g, 0,026 mole) dans 100 ml de méthanol. On ajoute 50 ml d'une solution aqueuse de HCI 2N. On chauffe le mélange à 50°C pendant 2 heures. On évapore le mélange sous vide à 40°C pour éliminer le méthanol. On verse le résidu dans 200 ml d'eau. On neutralise le mélange avec du bicarbonate de sodium. On extrait à l'acétate d'éthyle. On lave la phase organique à l'eau salée et la sèche sur Na₂SO₄. Le solvant est évaporé pour donner une huile jaune qui est purifiée sur silice (éluant : cyclohexane/acétate d'éthyle 60/40).
On obtient 5,6 g (rendement 61% en deux étapes) du produit recherché sous la forme d'une huile jaunâtre.
Spectre H¹ RMN (CDCl₃, 200MHz) : 7,27 (m, 5H) ; 5,35 (s, 2H) ; 4,55 (d, J=3Hz, 1H) ; 3,74 (m, 5H) ; 2,31 (d large, 1H) ; 2,06 (t, large, 1H) ; 0,00 (s, 9H).

### d) 4^{ème} étape : préparation du 5-(1,2-dihydroxy-éthyl)-4-hydroxy-3-triméthylsilanylméthoxy-5H-furan-2-one

On dissout le dérivé précédent (5,2 g, 0,015 mole) dans 100 ml d'acétate d'éthyle.
On ajoute 100 ml d'éthanol absolu. On ajoute ensuite 1 g de palladium sur charbon à 5%. On agite le mélange sous 12 bars d'hydrogène pendant 7 heures à température ambiante. On filtre le catalyseur.
On évapore le solvant pour obtenir 3,8 g (rendement 98%) du produit recherché sous la forme d'un solide blanc.
Spectre H¹ RMN (acétone-d₆, 200MHz) : 4,74 (d, J=2Hz, 1H) ; 3,82 (m, 1H) ; 3,71 (m, 2H) ; 3,63 (m, 2H) ; 0,00 (s, 9H).

### Exemple 3 : Préparation du 5-(1,2-dihydroxy-éthyl)-3-hydroxy-4-(3-triméthylsilanylpropyloxy)-5H-furan-2-one

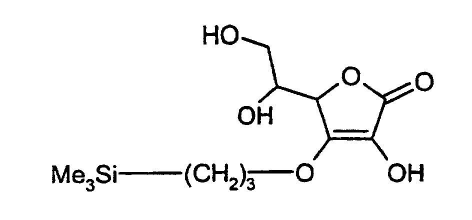

### a) 1^{ère} étape : préparation du 5-(2,2-diméthyl-[1,3]dioxolan-4-yl)-3-hydroxy-4-(3-triméthylsilanyl-propyloxy)-5H-furan-2-one

A un mélange de 5-(2,2-diméthyl-[1,3]dioxolan-4-yl)-3,4-dihydroxy-5H-furan-2-one (8,64 g, 0,04 mole) et de bicarbonate de sodium (5,04 g, 0,06 mole) dans 50 ml de diméthylsulfoxyde, sous azote, on ajoute du iodo-3-propyl triméthyl silane (14,5 g, 0,06 mole). On chauffe le mélange à 55°C pendant 24 heures. On le refroidit et on le verse dans 300 ml d'eau. On extrait à l'acétate d'éthyle. On lave la phase organique à l'eau salée et la sèche sur Na₂SO₄. On évapore le solvant.
On obtient une huile brune qui est purifiée sur silice (éluant : cyclohexane/acétate d'éthyle 75/25) pour donner 8,8 g (rendement 67%) de produit recherché sous la forme d'une huile légèrement jaune.
Spectre H¹ RMN (CDCl₃, 200MHz) : 4,53 (d, J=4Hz, 1H) ; 4,40 (t, J=7Hz, 2H) ; 4,25 (m, 1H) ; 3,98 (m, 2H) ; 1,63 (m, 2H) ; 0,52 (m, 2H) ; 0,00 (s, 9H).

### b) 2^{ème} étape : préparation du 5-(1,2-dihydroxy-éthyl)-3-hydroxy-4-(3-triméthylsilanylpropyloxy)-5H-furan-2-one

On dissout le produit précédent (8,89 g, 0,03 mole) dans 100 ml de méthanol. On ajoute 50 ml d'une solution aqueuse de HCI 2N. On chauffe le mélange à 50°C pendant 2 heures. On évapore le mélange sous vide à 40°C pour éliminer le méthanol. On verse le résidu dans 100 ml d'eau. On neutralise le mélange avec du bicarbonate de sodium. On extrait à l'acétate d'éthyle. On lave la phase organique à l'eau salée et la sèche sur Na₂SO₄. On évapore le solvant. L'huile jaunâtre obtenue est purifiée sur silice (éluant: cyclohexane/acétate d'éthyle 50/50).
On obtient 5,4 g (rendement 62%) du produit recherché sous la forme d'une huile incolore.
Spectre H¹ RMN (CDCl₃, 200MHz) : 4,65 (d, J=2Hz, 1H) ; 4,33 (m, 2H) ; 3,98 (m, 2H) ; 3,81 (m, 2H) ; 1,63 (m, 2H) ; 0,51 (m, 2H) ; 0,00 (s, 9H).

### Exemple 4 : Préparation du 5-(1,2-dihydroxy-éthyl)-4-hydroxy-3-(3-triméthylsilanylpropyloxy)-5H-furan-2-one

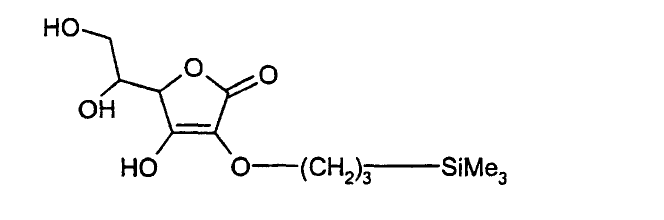

### a) 1^{ère} étape : préparation du 4-benzyloxy-5-(2,2-diméthyl-[1,3]dioxolan-4-yl)-3-(3-triméthylsilanyl-propyloxy)-5H-furan-2-one

A un mélange de 4-benzyloxy-5-(2,2-diméthyl-[1,3]dioxolan-4-yl)-3-hydroxy-5H-furan-2-one (14,8 g, 0,048 mole) et de carbonate de potassium (8,0 g, 0,058 mole), sous azote, dans 100 ml de diméthylsulfoxyde, on ajoute du iodo-3-propyl triméthylsilane (12,86 g, 0,053 mole). On agite le mélange à température ambiante pendant 22 heures. On le verse dans 300 ml d'eau. On extrait à l'acétate d'éthyle. On lave la phase organique à l'eau salée et la sèche sur Na₂SO₄.
Après évaporation du solvant, on obtient une huile orangée qui est purifiée sur silice (éluant : cyclohexane/acétate d'éthyle 5/1) pour donner 10,5 g (rendement 52%) de produit recherché sous la forme d'une huile incolore.
Spectre H¹ RMN (CDCl₃, 200MHz) : 7,39 (m, 5H) ; 5,50 (s, 2H) ; 4,55 (d, J=3Hz, 1H) ; 4,30 (m, 1H) ; 4,01 (m, 4H) ; 1,67 (m, 2H) ; 1,40 (s, 3H) ; 1,37 (s, 3H), 0,50 (m, 2H) ; 0,00 (s, 9H).

### b) 2^{ème} étape : préparation du 4-benzyloxy-5-(1,2-dihydroxy-éthyl)-3-(3-triméthylsilanylpropyloxy)-5H-furan-2-one

On dissout le produit précédent (9,4 g, 0,025 mole) dans 80 ml de méthanol. On ajoute 40 ml d'une solution aqueuse d' HCl 2N. On chauffe le mélange à 50°C pendant 2 heures. On évapore le mélange sous vide à 40°C pour éliminer le méthanol. On verse le résidu dans 200 ml d'eau. On neutralise le mélange avec du bicarbonate de sodium. On extrait à l'acétate d'éthyle. On lave la phase organique à l'eau salée et la sèche sur Na₂SO₄. Le solvant est évaporé pour donner une huile jaune qui est purifiée sur silice (éluant : cyclohexane/acétate d'éthyle 2/1). On obtient 8,0 g (rendement 94%) du produit recherché sous la forme d'une huile jaunâtre.
Spectre H¹ RMN (CDCl₃, 200MHz) : 7,39 (m, 5H) ; 5,50 (s, 2H), 4,71 (d, J=3Hz, 1H) ; 4,01 (m, 3H) ; 3,80 (m, 2H) ; 2,47 (d large, 1H) ; 2,21 (t large, 1H) ; 0,50 (m, 2H) ; 0,00 (s, 9H).

### c) 3^{ème} étape : préparation du 5-(1,2-dihydroxy-éthyl)-4-hydroxy-3-(3-triméthylsilanylpropyloxy)-5H-furan-2-one

On dissout le dérivé précédent (7,6 g, 0,02 mole) dans 100 ml d'acétate d'éthyle. On ajoute 100 ml d'éthanol absolu. On ajoute ensuite 1,2 g de palladium sur charbon à 5%. On agite le mélange sous 12 bars d'hydrogène pendant 7 heures à température ambiante. On filtre le catalyseur.
On évapore le solvant et l'on obtient 5,7 g (rendement 98%) du produit recherché sous la forme d'un solide blanc.

Spectre H¹ RMN (acétone-d₆, 200MHz) : 4,84 (d, J=2Hz, 1H) ; 3,96 (m, 3H), 3,89 (m, 2H) ; 1,65 (m, 2H) ; 0,54 (m, 2H) ; 0,00 (s, 9H).

### Exemple 5 : Crème solaire

On prépare une émulsion huile-dans-eau anti-solaire, comprenant :
- composé de l'exemple 1 1 g
- filtre UV (Mexoryl®XL) 4 g
- mélange d'alcool cétylstéarylique et d'alcool cétylstéarylique oxyéthyléné (33 OE) 80/20 (DEHSCONET 390 de TENSIA) 7 g
- mélange de mono et distéarate de glycérol (CERASYNTH SD de ISP) 2 g
- polydiméthylsiloxane (DC200 Fluid de DOW CORNING) 1,5 g
- benzoate d'alcools en C₁₂-C₁₅ (FINSOLV TN de FINETEX) 16 g
- glycérine 20 g
- eau déminéralisée qsp 100 g

### Exemple 6 : Gel conditionneur après-shampooing protecteur des cheveux

On prépare un gel conditionneur après-shampooing protecteur des cheveux, comprenant :
- composé de l'exemple 4 2 g
- polydiméthylsiloxane α, Ω-dihydroxylé/silicone volatile (Q2-1401 de DOW CORNING) 20 g
- copolymère réticulé acrylamide/acide 2-acrylamido-2-méthylpropane sulfonique (SEPIGEL 305 par SEPPIC) 1 g MA
- eau qsp 100 g

## Revendications

1. Composé silicié dérivé de l'acide ascorbique, **caractérisé par** le fait
- soit qu'il est constitué d'une chaîne siliconée comportant au moins une unité de formule (1) : 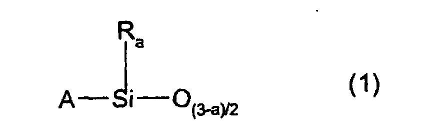
- soit qu'il est un silane répondant à la formule (2) suivante :
A-SiR^{'}₁R^{'}₂R^{'}₃ (2)
dans lesquelles :
- R désigne un radical hydrocarboné, linéaire, cyclique ou ramifié, saturé ou insaturé en C₁-C₃₀, éventuellement partiellement ou totalement halogéné, ou un groupe triméthylsilyloxy de formule -O-SiMe₃;
- a est égal à 1 ou 2,
- R'₁, R'₂, R'₃ , identiques ou différents, sont choisis parmi les radicaux alkyles linéaires ou ramifiés en C₁-C₈, les radicaux alcényles linéaires ou ramifiés en C₁-C₈, ou un groupe triméthylsilyloxy;
- A est un radical de formule (I) suivante : 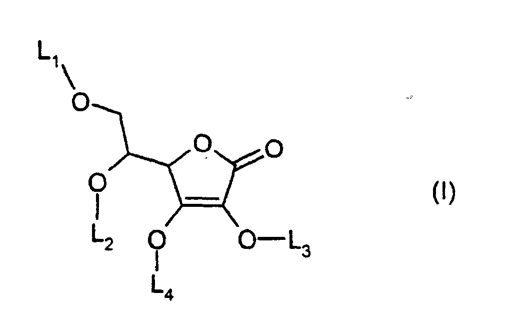
dans laquelle L₁, L₂, L₃ et L₄ représentent l'hydrogène ou un radical divalent de formule (a) ou (a') permettant l'accrochage du radical A sur la chaîne siliciée, sous réserve qu'au moins un des radicaux L₁, L₂, L₃ et L₄, de préférence un seul desdits radicaux L₁, L₂, L₃ et L₄, représente ledit radical divalent de formule (a) ou (a') suivante : 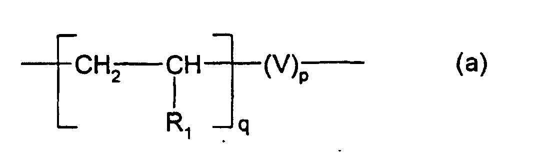 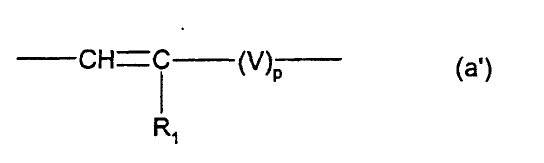 dans lesquelles :
- V est un radical hydrocarboné divalent en C₁-C₆ linéaire ou ramifié, saturé ou insaturé, éventuellement substitué par un radical hydroxyle ou un radical alcoxy en C₂-C₈, linéaire ou ramifié, saturé ou insaturé;
- R₁ représente un atome d'hydrogène, un radical hydroxyle ou un radical hydrocarboné en C₁-C₈, linéaire ou ramifié, saturé ou insaturé;
- p est 0 ou 1, q est 0 ou 1, étant donné que p + q est différent de 0.

2. Composé selon la revendication 1, dans lequel la chaîne siliconée comporte au moins une unité de formule (1a) : 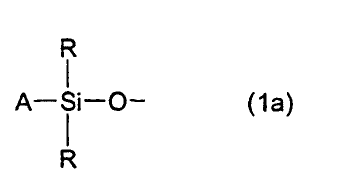 et/ou au moins une unité de formule (1b) : 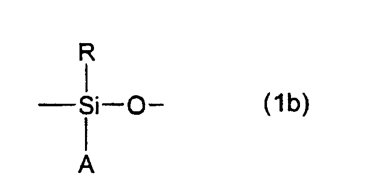 et éventuellement au moins une autre unité, par exemple de type (di-)-alkylsiloxane de formule (1c) :

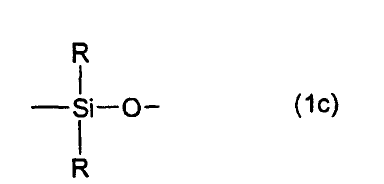

3. Composé selon l'une des revendications précédentes, répondant à l'une des formules (3) ou (4) suivantes : 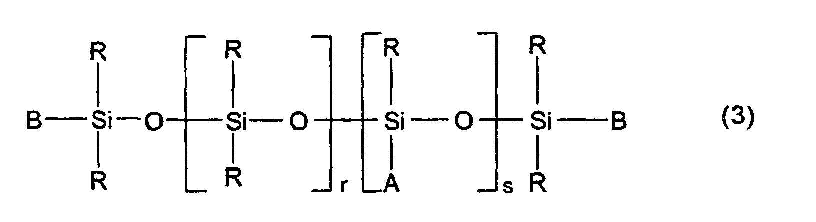 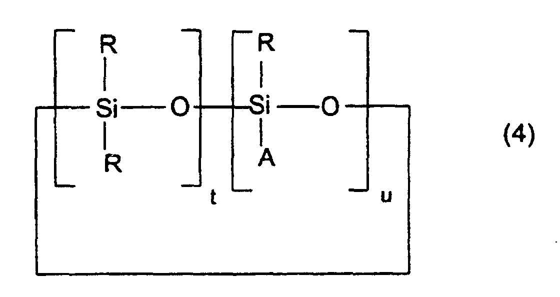 dans lesquelles :
- les radicaux B, identiques ou différents, sont choisis parmi les radicaux R et A,
- r est un nombre entier compris entre 0 et 50 inclus, de préférence entre 0 et 5,
- s est un nombre entier compris entre 0 et 20 inclus, de préférence choisi parmi 0, 1 ou 2, sous réserve que si s=0, alors au moins l'un des deux radicaux B, représente A,
- u est un nombre entier compris entre 1 et 6 inclus, de préférence choisi parmi 1 ou 2,
- t est un nombre entier entre 0 et 9 inclus, de préférence choisi parmi 2, 3 ou 4,
- étant donné que t + u est compris entre 3 et 10 inclus, de préférence vaut 3, 4, 5 ou 6.

4. Composé selon l'une des revendications précédentes, dans lequel les radicaux R, identiques ou différents, sont choisis parmi les radicaux hydrocarbonés en C₁-C₁₈, linéaires, cycliques ou ramifiés, saturés ou insaturés, et les radicaux hydrocarbonés en C₁-C₈, linéaires ou ramifiés, saturés ou insaturés, partiellement halogénés, notamment fluorés; et plus particulièrement parmi les radicaux alkyles en C₁-C₁₀, linéaires ou ramifiés, notamment méthyle; le radical phényle; les radicaux alkyles fluorés en C₁-C₈, linéaires ou ramifiés, notamment 3,3,3-trifluoropropyle.

5. Composé selon l'une des revendications précédentes, dans lequel les radicaux R'₁, R'₂, R'₃, identiques ou différents, sont choisis parmi les radicaux alkyles linéaires ou ramifiés en C₁-C₆ , notamment méthyle ou éthyle, et le groupe triméthylsilyloxy.

6. Composé selon l'une des revendications précédentes, dans lequel les radicaux divalents correspondant aux formules (a) ou (a') sont choisis parmi les radicaux hydrocarbonés, linéaires ou ramifiés, saturés ou insaturés, éventuellement hydroxylés, divalents en C₁-C₆ tels que les radicaux méthylène (-CH₂-), éthylène (-CH₂-CH₂-), propylène (-CH₂-CH₂-CH₂-), n-butylène (-CH₂-CH₂-CH₂-CH₂-), iso-butylène (-CH₂-CH(CH₃)-CH₂-), les radicaux -CH=CH-CH₂-, -CH=C(CH₃)-CH₂-, -CH=CH-CH(CH₃)- et -CH₂-CH(OH)-CH₂-.

7. Composé selon l'une des revendications précédentes, choisi parmi :
- la 5-(1,2-dihydroxy-éthyl)-3-hydroxy-4-(3-[1,3,3,3-tetraméthyl-1-[(triméthylsilyl) oxy]disiloxanyl]propyloxy)-5H-furan-2-one,
- la 5-(1,2-dihydroxy-éthyl)-4-hydroxy-3-(3-[1,3,3,3-tetraméthyl-1-[(triméthylsilyl) oxy]disiloxanyl]propyloxy)-5H-furan-2-one,
- la 5-(1,2-dihydroxy-éthyl)-3-hydroxy-4-[2-méthyl-3-[1,3,3,3-tetraméthyl-1-[(triméthylsilyl)oxy]disiloxanyl]propyloxy]-5H-furan-2-one,
- la 5-(1,2-dihydroxy-éthyl)-4-hydroxy-3-[2-méthyl-3-[1,3,3,3-tetraméthyl-1-[(triméthylsilyl)oxy]disiloxanyl]propyloxy]-5H-furan-2-one,
- la 5-(1,2-dihydroxy-éthyl)-4-[3-[3-[2-méthyl-3-[1,3,3,3-tetraméthyl-1-[(triméthylsilyl)oxy]disiloxanyl]propyloxy]-2-hydroxy-propyloxy]-3-hydroxy-5H-furan-2-one,
- la 5-(1,2-dihydroxy-éthyl)-3-[3-[3-[2-méthyl-3-[1,3,3,3-tetraméthyl-1-[(triméthylsilyl)oxy]disiloxanyl]propyloxy]-2-hydroxy-propyloxy]-4-hydroxy-5H-furan-2-one,
- la 5-(1,2-dihydroxy-éthyl)-3-hydroxy-4-triméthylsilanylméthoxy-5H-furan-2-one,
- la 5-(1,2-dihydroxy-éthyl)-4-hydroxy-3-(3-triméthylsilanylméthoxy)-5H-furan-2-one,
- la 5-(1,2-dihydroxy-éthyl)-3-hydroxy-4-(3-triméthylsilanylpropoxy)-5H-furan-2-one, et
- la 5-(1,2-dihydroxy-éthyl)-4-hydroxy-3-(3-triméthylsilanylpropoxy)-5H-furan-2-one

8. Procédé de préparation des composés de formules (2) à (4), par hydrosilylation du dérivé siloxanique ou silanique correspondant représenté par l'une des formules (5) à (7) suivantes :
H-SiR^{'}₁R^{'}₂R^{'}₃ (5) 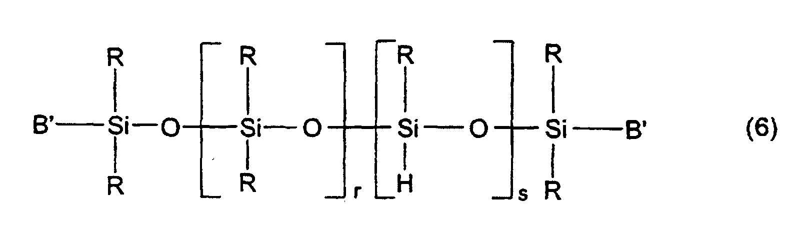 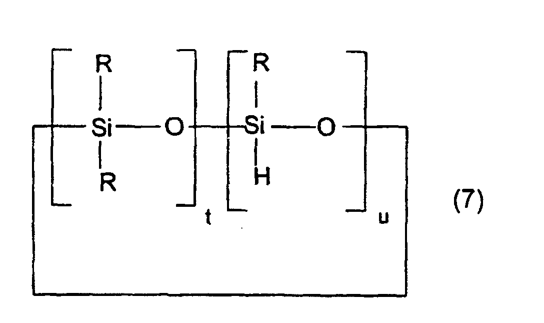
dans lesquelles :
- R'₁, R'₂, R'₃, R, r, s, t et u ont la signification donnée ci-dessus,
- B', identiques ou différents, sont choisis parmi les radicaux R et un atome d'hydrogène, sous réserve que si s=0, alors au moins l'un des deux radicaux B' représente H;
sur un dérivé organique d'acide ascorbique choisi parmi les composés de formule (I') suivante : 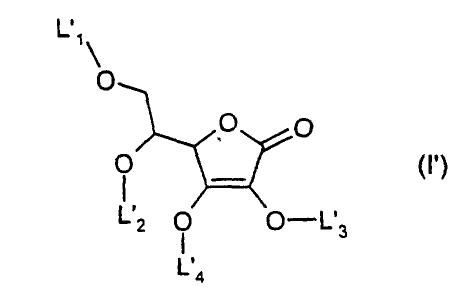 dans laquelle L'₁, L'₂, L'₃ et L'₄ représentent des groupements benzyloxy ou répondent à l'une des deux formules (b) et (b') suivantes : 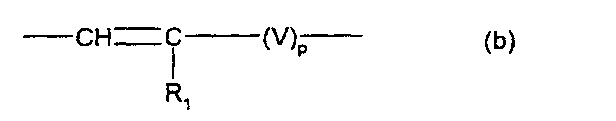 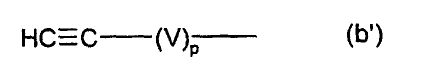 dans lesquelles R₁, V et p ont les mêmes significations que ci-dessus,
les radicaux L'₁ et L'₂ pouvant en outre former ensemble avec le reste d'acide ascorbique un cycle méthylène dioxy substitué par au moins un groupement alkyle en C₁₋₆ ou un groupement phényle, notamment par un groupement méthyle, éthyle ou phényle, voire par deux groupements alkyles ou phényles;
sous réserve qu'au moins un des radicaux L'₁, L'₂, L'₃ et L'₄ représente le radical (b) ou (b');
suivie d'une déprotection du composé obtenu, par exemple par hydrolyse acide des isopropylidènes et/ou débenzylation par hydrogénation catalytique.

9. Procédé de préparation des dérivés silaniques de formule (2) par réaction d'un dérivé de formule (I"') : 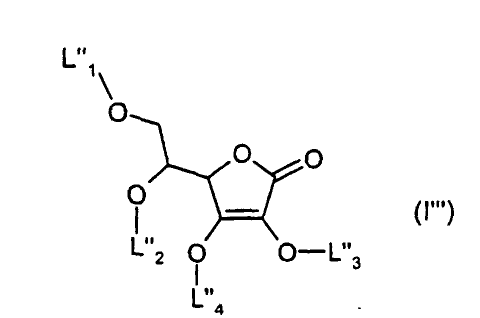 dans laquelle L"₁, L"₂, L"₃ et L"₄ représentent des groupements benzyloxy, un atome d'hydrogène,
les radicaux L"₁ et L"₂ pouvant en outre former ensemble avec le reste d'acide ascorbique un cycle méthylène dioxy substitué par au moins un groupement alkyle en C₁₋₆ ou un groupement phényle, notamment par un groupement méthyle, éthyle ou phényle, voire par deux groupements alkyles ou phényles;
sous réserve qu'au moins un des radicaux L"₁, L"₂, L"₃ et L"₄ représente l'hydrogène,
avec un dérivé silanique de formule (8) suivante : 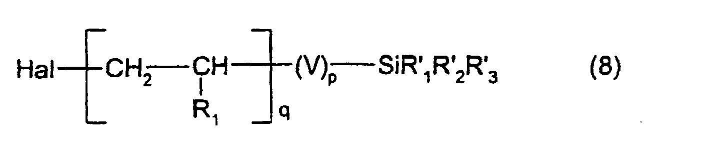 dans laquelle Hal représente un halogène et plus particulièrement le chlore ou l'iode et les radicaux R₁, R'₁, R'₂, R'₃, V, p et q ont les mêmes significations que ci-dessus.

10. Composition notamment cosmétique ou pharmaceutique, comprenant un milieu cosmétiquement ou pharmaceutiquement acceptable et au moins un composé selon l'une des revendications 1 à 7.

11. Composition selon la revendication 10, dans laquelle le composé est présent à raison de 0,1% à 10% en poids, de préférence entre 0,5% et 5% en poids, par rapport au poids total de la composition.

12. Composition selon l'une des revendications 10 à 11, se présentant sous la forme d'une composition à appliquer sur la peau du corps et/ou du visage, et/ou sur les cheveux, cils, sourcils et/ou ongles.

13. Composition selon l'une des revendications 10 à 12, se présentant sous la forme d'une composition protectrice de l'épiderme humain ou des cheveux contre les UV (compositions antisolaires); d'une composition cosmétique pour la protection ou de traitement ou soin des cheveux, notamment sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux; d'une composition de soin de la peau du corps et/ou du visage, telle que crème de traitement de l'épiderme, crème de jour, de nuit, crème anti-rides, crème hydratante, crème pour les mains ou les pieds; d'une composition de maquillage des cils, des sourcils, des cheveux, du corps ou du visage, telle que fond de teint, rouge à lèvres, fards à paupières, fards à joues, ligneur encore appelé "eye-liner", mascara, gel colorant, vernis à ongles.

14. Utilisation d'au moins un composé selon l'une des revendications 1 à 7, pour la préparation d'une composition pharmaceutique destinée à traiter le stress oxydant et/ou traiter les effets de l'exposition aux rayonnements ionisants ou solaires, et/ou prévenir le vieillissement notamment de la peau, des cheveux, des cils, des sourcils et/ou des ongles, et/ou traiter les effets de l'utilisation de certains médicaments générateurs de radicaux libres.

## Patentansprüche

1. Siliciumhaltige, von Ascorbinsäure abgeleitete Verbindung, **dadurch gekennzeichnet, dass**
- sie entweder aus einer Siliconkette besteht, die mindestens eine Einheit der Formel (1) enthält: 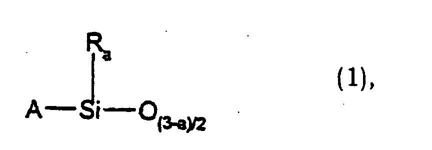
- oder ein Silan ist, das der folgenden Formel (2) entspricht:
A-SiR^{'}₁R^{'}₂R^{'}₃ (2),
worin:
- die Gruppe R eine gesättigte oder ungesättigte, geradkettige, cyclische oder verzweigte Kohlenwasserstoffgruppe mit 1 bis 30 Kohlenstoffatomen, die gegebenenfalls ganz oder teilweise halogeniert ist, oder eine Trimethylsilyloxygruppe der Formel -O-SiMe₃ bedeutet;
- a 1 oder 2 bedeutet,
- die Gruppen R'₁, R'₂, R'₃, die gleich oder verschieden sind, unter den geradkettigen oder verzweigten C₁₋₈-Alkylgruppen, den geradkettigen oder verzweigten C₁₋₈-Alkenylgruppen oder Trimethylsilyloxy ausgewählt sind;
- A eine Gruppe der folgenden Formel (I) ist: 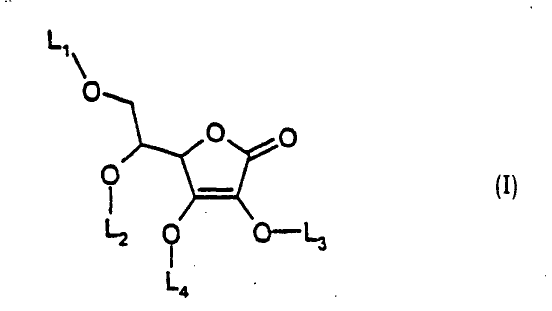
worin L₁, L₂, L₃ und L₄ Wasserstoff oder eine zweiwertige Gruppe der Formel (a) oder (a') bedeuten, mit der die Gruppe A an die siliciumhaltige Kette gebunden werden kann, mit der Maßgabe, dass mindestens eine der Gruppen L₁, L₂, L₃ und L₄ und vorzugsweise nur eine der Gruppen L₁, L₂, L₃ und L₄ eine zweiwertige Gruppe der folgenden Formel (a) oder (a') bedeutet: 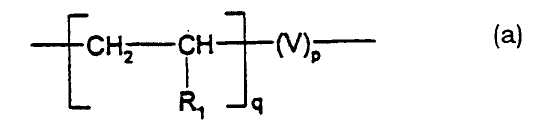 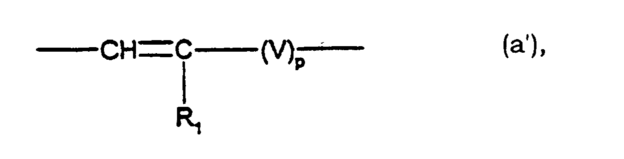 worin bedeuten:
- V eine geradkettige oder verzweigte, gesättigte oder ungesättigte, zweiwertige C₁₋₆-Kohlenwasserstoffgruppe, die gegebenenfalls mit einer Hydroxygruppe oder einer geradkettigen oder verzweigten, gesättigten oder ungesättigten C₂₋₈-Alkoxygruppe substituiert ist,
- R₁ ein Wasserstoffatom, eine Hydroxygruppe oder eine geradkettige oder verzweigte, gesättigte oder ungesättigte C₁₋₈-Kohlenwasserstoffgruppe,
- p 0 oder 1, q 0 oder 1, mit der Maßgabe, dass p + q von 0 verschieden ist.

2. Verbindung nach Anspruch 1, wobei die Siliconkette mindestens eine Einheit der Formel (1a) 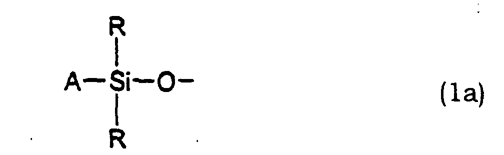 und/oder mindestens eine Einheit der Formel (1b): 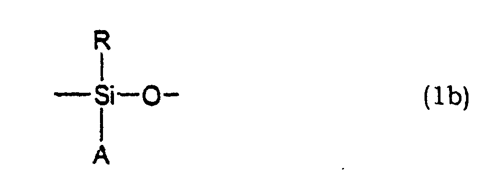 und gegebenenfalls mindestens eine weitere Einheit beispielsweise vom Typ (Di)alkylsiloxan der Formel (1c) enthält:

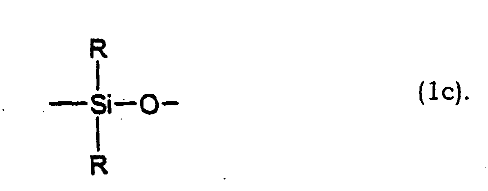

3. Verbindung nach einem der vorhergehenden Ansprüche, die einer der folgenden Formeln (3) oder (4) entspricht: 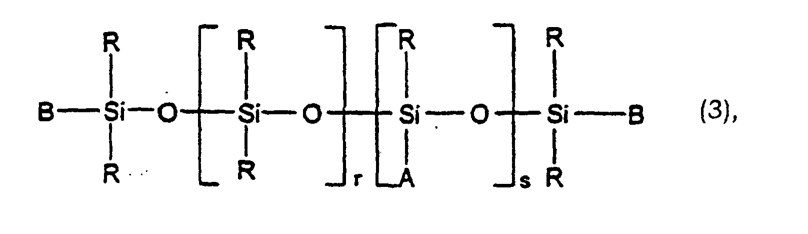 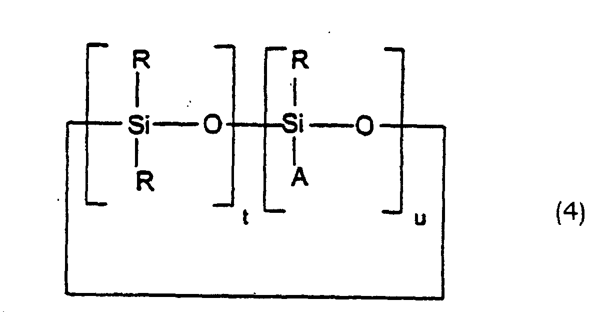 worin:
- die Gruppen B, die gleich oder verschieden sind, unter den Gruppen R und A ausgewählt sind,
- r 0 oder eine ganze Zahl im Bereich von 1 bis 50 und vorzugsweise 0 oder ein ganze Zahl im Bereich 1 bis 5 bedeutet,
- s 0 oder eine ganze Zahl im Bereich von 1 bis 20 und vorzugsweise im 0, 1 oder 2 bedeutet, mit der Maßgabe, dass mindestens eine der beiden Gruppen B A bedeutet, wenn s = 0,
- u eine ganze Zahl im Bereich von 1 bis 6 und vorzugsweise 1 oder 2 bedeutet,
- t 0 oder eine ganze Zahl von 1 bis 9 bedeutet und vorzugsweise unter 2, 3 oder 4 ausgewählt ist,
- mit der Maßgabe, dass t + u im Bereich von 3 bis 10 liegt und vorzugsweise 3, 4, 5 oder 6 bedeutet.

4. Verbindung nach einem der vorhergehenden Ansprüche, wobei die Gruppen R, die gleich oder verschieden sind, unter den geradkettigen, cyclischen oder verzweigten, gesättigten oder ungesättigten C₁₋ ₁₈-Kohlenwasserstoffgruppen und den geradkettigen oder verzweigten, gesättigten oder ungesättigten, teilweise halogenierten und insbesondere fluorierten C₁₋₈-Kohlenwasserstoffgruppen ausgewählt sind, insbesondere unter den geradkettigen oder verzweigten C₁₋₁₀-Alkylgruppen, besonders Methyl; Phenyl; und den geradkettigen oder verzweigten fluorierten C₁₋₈-Alkylgruppen und insbesondere 3,3,3-Trifluorpropyl.

5. Verbindung nach einem der vorhergehenden Ansprüche, wobei die Gruppen R'₁, R'₂, R'₃, die gleich oder verschieden sind, unter den geradkettigen oder verzweigten C₁₋₆-Alkylgruppen, besonders Methyl oder Ethyl, und der Trimethylsilyloxygruppe ausgewählt sind.

6. Verbindung nach einem der vorhergehenden Ansprüche, wobei die zweiwertigen Verbindungen der Formeln (a) oder (a') unter den geradkettigen oder verzweigten, gesättigten oder ungesättigten, gegebenenfalls hydroxylierten, zweiwertigen C₁₋₆-Kohlenwasserstoffgruppen ausgewählt sind, beispielsweise den Gruppen Methylen (-CH₂-), Ethylen (-CH₂-CH₂-), Propylen (-CH₂-CH₂-CH₂-), n-Butylen (-CH₂-CH₂-CH₂-CH₂-), Isobutylen (-CH₂-CH(CH₃)-CH₂-) und den Gruppen -CH=CH-CH₂-, -CH=C(CH₃)-CH₂, -CH=CH-CH(CH₃)- und -CH₂-CH(OH)-CH₂-.

7. Verbindung nach einem der vorhergehenden Ansprüche, die ausgewählt ist unter:
- 5-(1,2-Dihydroxy-ethyl)-3-hydroxy-4-(3-[1,3,3,3-tetramethyl-1-[(trimethylsilyl)oxy]disiloxanyl]propyloxy)-5H-furan-2-on,
- 5-(1,2-Dihydroxy-ethyl)-4-hydroxy-3-(3-[1,3,3,3-tetramethyl-1-[(trimethylsilyl)oxy]disiloxanyl]propyloxy)-5H-furan-2-on,
- 5-(1,2-Dihydroxy-ethyl)-3-hydroxy-4-[2-methyl-3-[1,3,3,3-tetramethyl-1-[(trimethylsilyl)oxy]disiloxanyl]propyloxy]-5H-furan-2-on,
- 5-(1,2-Dihydroxy-ethyl)-4-hydroxy-3-[2-methyl-3-[1,3,3,3-tetramethyl-1-[(trimethylsilyl)oxy]disiloxanyl]propyloxy] -5H-furan-2-on,
- 5-(1,2-Dihydroxy-ethyl)-4-[3-[3-[2-methyl-3-[1,3,3,3-tetramethyl-1-[(trimethylsilyl)oxy]disiloxanyl]propyloxy]-2-hydroxy-propyloxy]-3-hydroxy-5H-furan-2-on,
- 5-(1,2-Dihydroxy-ethyl)-3-[3-[3-[2-methyl-3-[1,3,3,3-tetramethyl-1-[(trimethylsilyl)oxy]disiloxanyl]propyloxy]-2-hydroxy-propyloxy]-4-hydroxy-5H-furan-2-on,
- 5-(1,2-Dihydroxy-ethyl)-3-hydroxy-4-trimethylsilanylmethoxy-5Hfuran-2-on,
- 5-(1,2-Dihydroxy-ethyl)-4-hydroxy-3-(3-trimethylsilanylmethoxy)-5H-furan-2-on,
- 5-(1,2-Dihydroxy-ethyl)-3-hydroxy-4-(3-trimethylsilanylpropyloxy)-5H-furan-2-on, und
- 5-(1,2-Dihydroxy-ethyl)-4-hydroxy-3-(3-trimethylsilanylpropyloxy)-5H-furan-2-on.

8. Verfahren zur Herstellung von Verbindungen der Formeln (2) bis (4) durch Hydrosilylierung des entsprechenden Siloxanderivats oder Silanderivats einer der folgenden Formeln (5) bis (7):
H-SiR^{'}₁R^{'}₂R^{'}₃ (5), 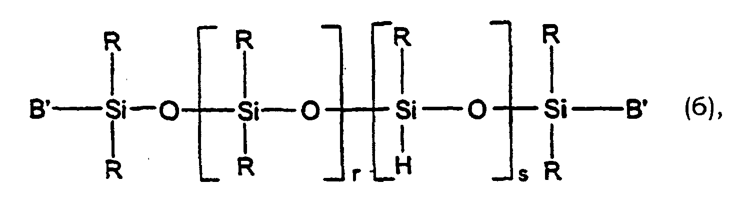 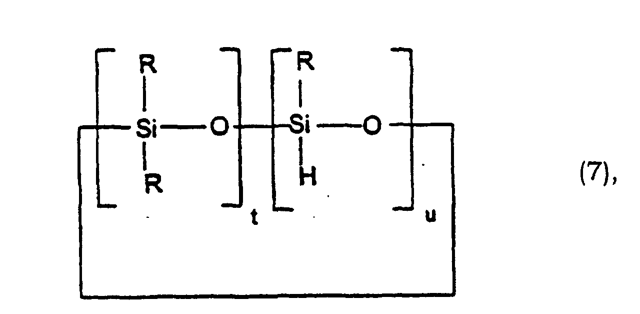
worin:
- R'₁, R'₂, R'₃, r, s, t und u die oben angegebenen Bedeutungen haben und,
- die Gruppen B', die identisch oder voneinander verschieden sind, unter den Gruppen R und Wasserstoff ausgewählt sind, mit der Maßgabe, dass mindestens eine der beiden Gruppen B' H bedeutet, wenn s = 0;
mit einem organischen Derivat von Ascorbinsäure, das unter den Verbindungen der folgenden Formel (I') ausgewählt ist 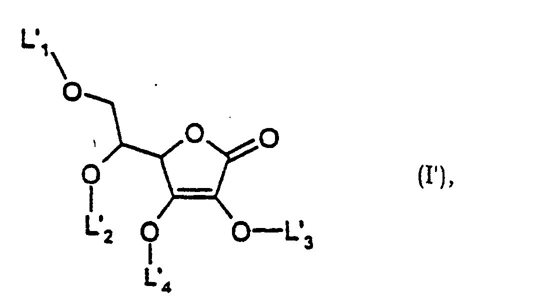 worin L'₁, L'₂, L'₃ und L'₄ Benzyloxygruppen bedeuten oder einer der beiden folgenden Formeln (b) und (b') entsprechen: 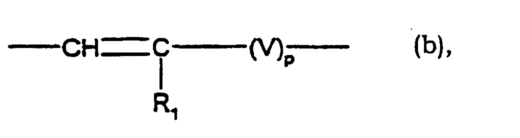 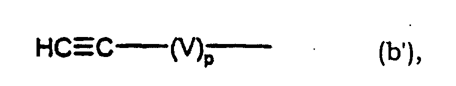 worin R₁, V und p die oben angegebenen Bedeutungen aufweisen, wobei die Gruppen L'₁ und L'₂ ferner gemeinsam mit dem Ascorbinsäurerest einen Metyhlendioxyring bilden können, der mit mindestens einer C₁₋₆-Alkylgruppe oder einer Phenylgruppe, insbesondere Methyl, Ethyl oder Phenyl, oder sogar zwei Alkyl- oder Phenylgruppen substituiert ist; mit der Maßgabe, dass mindestens eine der Gruppen L'ᵢ, L'₂, L'₃ und L'₄ die Gruppe (b) oder (b') bedeutet,
wobei die Schutzgruppe der erhaltenen Verbindung anschließend entfernt wird, beispielsweise durch saure Hydrolyse von Isopropylidenen und/oder Debenzylierung durch katalytische Hydrierung.

9. Verfahren zur Herstellung von Silanderivaten der Formel (2) durch Umsetzung eines Derivats der Formel (I'''): 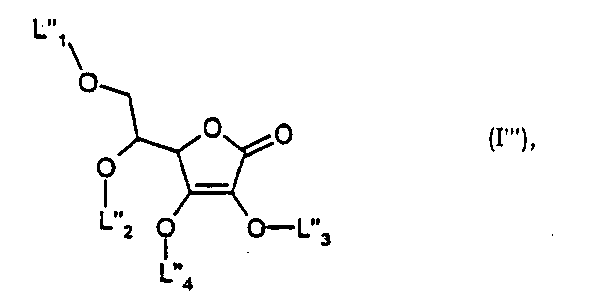 worin L"₁, L"₂, L"₃ und L"₄ Benzyloxygruppen oder ein Wasserstoffatom bedeuten, wobei die Gruppen L"₁ und L"₂ ferner gemeinsam mit dem Ascorbinsäurerest einen Methylendioxyring bilden können, der mit mindestens einer C₁₋₆-Alkylgruppe oder einer Phenylgruppe, insbesondere mit Methyl, Ethyl oder Phenyl, oder sogar zwei Alkyl- oder Phenylgruppen substituiert ist;
mit der Maßgabe, dass mindestens eine der Gruppen L"₁, L"₂, L"₃ und L"₄ Wasserstoff bedeutet,
mit einem Silanderivat der folgenden Formel (8): 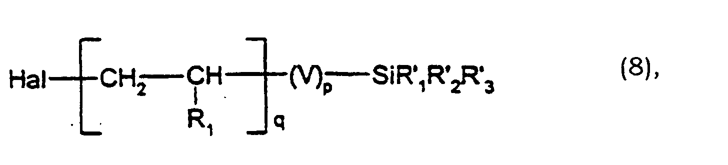 worin Hal Halogen und insbesondere Chlor oder Iod bedeutet und die Gruppen R₁, R'₁, R'₂, R'₃, V, p und q die oben angegebenen Bedeutungen aufweisen.

10. Zusammensetzung, insbesondere kosmetische oder pharmazeutische Zusammensetzung, die ein kosmetisch oder pharmazeutisch akzeptables Medium und mindestens eine Verbindung nach einem der Ansprüche 1 bis 7 enthält.

11. Zusammensetzung nach Anspruch 10, wobei die Verbindung in einer Menge von 0,1 bis 10 Gew.-% und vorzugsweise 0,5 bis 5 Gew.-%, bezogen auf das Gesamtgewicht der Zusammensetzung enthalten ist.

12. Zusammensetzung nach einem der Ansprüche 10 bis 11, die in Form einer Zusammensetzung vorliegt, die auf die Haut des Körpers und/oder des Gesichts und/oder die Haare, die Wimpern, die Augenbrauen und/oder die Nägel aufgetragen werden soll.

13. Zusammensetzung nach einem der Ansprüche 10 bis 12, die in Form einer Zusammensetzung zum Schutz der menschlichen Epidermis oder der Haare gegen UV-Strahlung (Sonnenschutzmittel); in Form einer kosmetischen Zusammensetzung zum Schutz oder zur Behandlung oder zur Pflege der Haare, insbesondere als Haarwaschmittel, Lotion, Gel oder Zusammensetzung, die ausgespült wird, vor oder nach einer Haarwäsche, vor oder nach einer Färbung oder Entfärbung, vor, während oder nach einer dauerhaften Verformung oder Entkräuselung aufgetragen wird, Lotion oder Gel zum Frisieren oder zur Behandlung, Lotion oder Gel zum Fönen oder für Wasserwellen, Haarlack, Zusammensetzung für Dauerwellen oder zum Entkräuseln, Zusammensetzung zum Färben oder Entfärben der Haare; in Form einer Zusammensetzung zur Pflege der Haut des Körpers und/oder des Gesichts, beispielsweise als Creme zur Behandlung der Epidermis, Tagescreme, Nachtcreme, Antifaltencreme, hydratisierende Creme, Creme für die Hände oder die Füße; und in Form einer Zusammensetzung zum Schminken der Wimpern, der Augenbrauen, der Haare, des Körpers oder des Gesichts, beispielsweise als Makeup, Lippenstift, Lidschatten, Wangenrouge, Lidstrichstift, der auch als Eyeliner bezeichnet wird, Mascara, färbendes Gel oder Nagellack, vorliegt.

14. Verwendung mindestens einer Verbindung nach einem der Ansprüche 1 bis 7 zur Herstellung einer pharmazeutischen Zusammensetzung, die dazu vorgesehen ist, oxidativen Stress zu behandeln und/oder die Wirkungen der Exposition gegenüber ionisierender Strahlung oder Sonnenlicht zu behandeln und/oder Alterung insbesondere der Haut, der Haare, der Wimpern, der Augenbrauen und/oder Nägel vorzubeugen und/oder die Wirkungen der Verwendung verschiedener Arzneimittel, die freie Radikale bilden, zu behandeln.

## Claims

1. Silicon-containing compound derived from ascorbic acid, **characterized in that**
- either it consists of a silicone-containing chain comprising at least one unit of formula (1) : 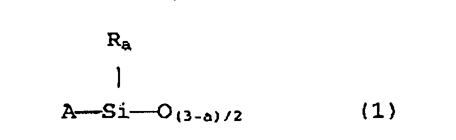
- or it is a silane corresponding to the following formula (2) :
A-SiR'₁R'₂R'₃ (2)
in which:
- R denotes a linear, cyclic or branched, saturated or unsaturated, optionally partially or completely halogenated, C₁-C₃₀ hydrocarbon radical or a trimethylsilyloxy group of formula -O-SiMe₃;
- a is equal to 1 or 2,
- R'₁, R'₂, R'₃, which are identical or different, are chosen from linear or branched C₁-C₈ alkyl radicals, linear or branched C₁-C₈ alkenyl radicals, or a trimethylsilyloxy group;
- A is a radical of the following formula (I) : 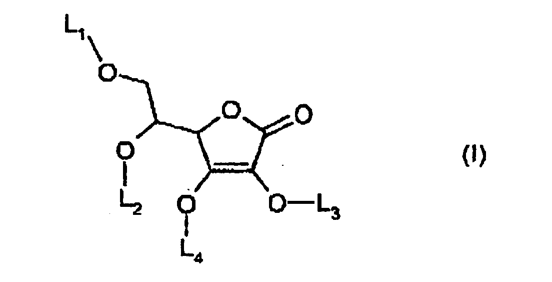 in which L₁, L₂, L₃ and L₄ represent hydrogen or a divalent radical of formula (a) or (a') allowing the attachment of the radical A onto the silicon-containing chain, with the proviso that at least one of the radicals L₁, L₂, L₃ and L₄, preferably only one of the said radicals L₁, L₂, L₃ and L₄, represents the said divalent radical of the following formula (a) or (a'): 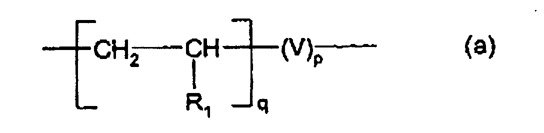 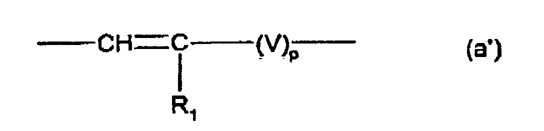 in which:
- V is a linear or branched, saturated or unsaturated, divalent C₁-C₆ hydrocarbon radical optionally substituted with a hydroxyl radical or a linear or branched, saturated or unsaturated C₂-C₈ alkoxy radical;
- R₁ represents a hydrogen atom, a hydroxyl radical or a linear or branched, saturated or unsaturated C₁-C₈ hydrocarbon radical;
- p is 0 or 1, q is 0 or 1, it being understood that p + q is different from 0.

2. Compound according to Claim 1, in which the silicone-containing chain comprises at least one unit of formula (1a) : 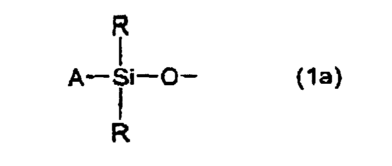 and/or at least one unit of formula (1b) : 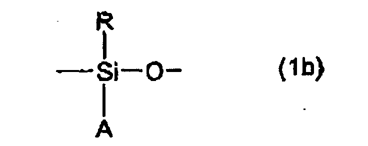 and optionally at least one other unit, for example of the (di-)alkylsiloxane type of formula (1c):

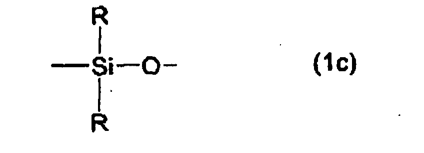

3. Compound according to one of the preceding claims, corresponding to one of the following formulae (3) or (4): 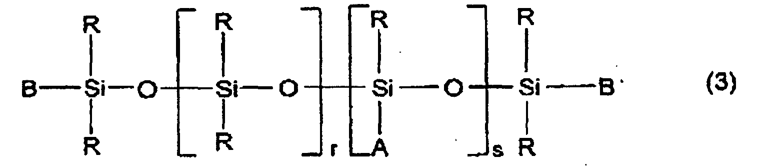 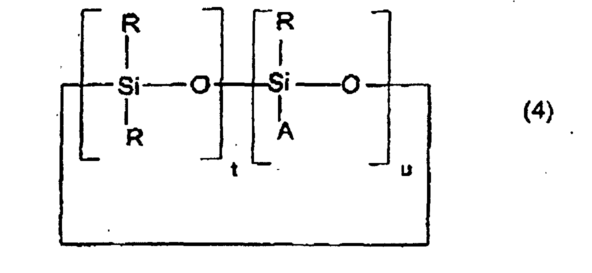 in which:
- the radicals B, which are identical or different, are chosen from the radicals R and A,
- r is an integer between 0 and 50 inclusive, preferably between 0 and 5,
- s is an integer between 0 and 20 inclusive, preferably chosen from 0, 1 or. 2, with the proviso that if s = 0, then at least one of the two radicals B represents A,
- u is an integer between 1 and 6 inclusive, preferably chosen from 1 or 2,
- t is an integer between 0 and 9 inclusive, preferably chosen from 2, 3 or 4,
- it being understood that t + u is between 3 and 10 inclusive, and is preferably equal to 3, 4, 5 or 6.

4. Compound according to one of the preceding claims, in which the radicals R, which are identical or different, are chosen from linear, cyclic or branched, saturated or unsaturated C₁-C₁₈ hydrocarbon radicals and linear or branched, saturated or unsaturated, C₁-C₈ hydrocarbon radicals, partially halogenated, in particular fluorinated; and more particularly from linear or branched C₁-C₁₀ alkyl radicals, in particular methyl; the phenyl radical; linear or branched, fluorinated C₁-C₈ alkyl radicals, in particular 3,3,3-trifluoropropyl.

5. Compound according to one of the preceding claims, in which the radicals R'₁, R'₂, R'₃, which are identical or different, are chosen from linear or branched C₁-C₆ alkyl radicals, in particular methyl or ethyl radicals, and the trimethylsilyloxy group.

6. Compound according to one of the preceding claims, in which the divalent radicals corresponding to the formulae (a) or (a') are chosen from linear or branched, saturated or unsaturated, optionally hydroxylated, divalent C₁-C₆ hydrocarbon radicals such as the methylene (-CH₂-), ethylene (-CH₂-CH₂-), propylene (-CH₂-CH₂-CH₂-), n-butylene (-CH₂-CH₂-CH₂-CH₂-) or isobutylene (-CH₂-CH(CH₃)-CH₂-) radicals, and the radicals -CH=CH-CH₂-, -CH=C(CH₃) -CH₂-, -CH=CH-CH(CH₃)-and CH₂-CH(OH)-CH₂-.

7. Compound according to one of the preceding claims, chosen from:
- 5-(1,2-dihydroxyethyl)-3-hydroxy-4-(3-[1,3,3,3-tetramethyl-1-[(trimethylsilyl)oxy]disiloxanyl]-propyloxy)-5H-furan-2-one,
- 5-(1,2-dihydroxyethyl)-4-hydroxy-3-(3-[1,3,3,3-tetramethyl-1-[(trimethylsilyl)oxy]disiloxanyl]-propyloxy)-5H-furan-2-one,
- 5-(1,2-dihydroxyethyl)-3-hydroxy-4-[2-methyl-3-[1,3,3,3-tetramethyl-1-[(trimethylsilyl)oxy]-disiloxanyl]propyloxy]-5H-furan-2-one,
- 5-(1,2-dihydroxyethyl)-4-hydroxy-3-[2-methyl-3-[1,3,3,3-tetramethyl-1-[(trimethylsilyl)oxy]-disiloxanyl]propyloxy]-5H-furan-2-one,
- 5-(1,2-dihydroxyethyl)-4-[3-[3-[2-methyl-3-[1,3,3,3-tetramethyl-1-[(trimethylsilyl)oxy]disiloxanyl]-propyloxy]-2-hydroxy-propyloxy]-3-hydroxy-5H-furan-2-one,
- 5-(1,2-dihydroxyethyl)-3-[3-[3-[2-methyl-3-[1,3,3,3-tetramethyl-1-[(trimethylsilyl)oxy]disiloxanyl]-propyloxy]-2-hydroxy-propyloxy]-4-hydroxy-5H-furan-2-one,
- 5-(1,2-dihydroxyethyl)-3-hydroxy-4-trimethylsilanylmethoxy-5H-furan-2-one,
- 5- (1,2-dihydroxyethyl)-4-hydroxy-3- (3-trimethylsilanylmethoxy)-5H-furan-2-one,
- 5-(1,2-dihydroxyethyl)-3-hydroxy-4-(3-trimethylsilanylpropoxy)-5H-furan-2-one, and
- 5-(1,2-dihydroxyethyl)-4-hydroxy-3-(3-trimethylsilanylpropoxy)-5H-furan-2-one.

8. Method of preparing the compounds of formulae (2) to (4) by hydrosilylation of the corresponding siloxane or silane derivative represented by one of the following formulae (5) to (7):
H-SiR'₁R'₂R'₃ (5) 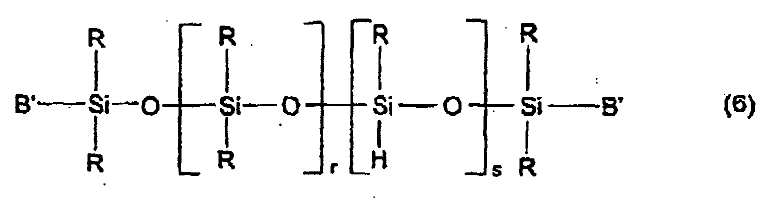 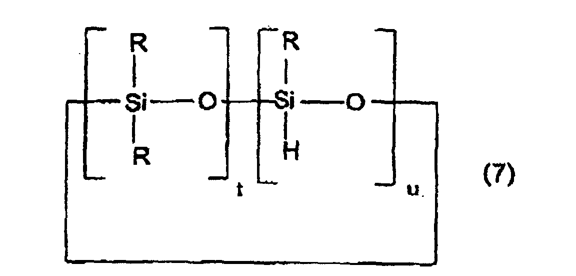
in which:
- R'₁, R'₂, R'₃, R, r, s, t and u have the meaning given above,
- B', which are identical or different, are chosen from the radicals R and a hydrogen atom, with the proviso that if s = 0, then at least one of the two radicals B' represents H;
on an organic derivative of ascorbic acid chosen from the compounds of the following formula (I'): 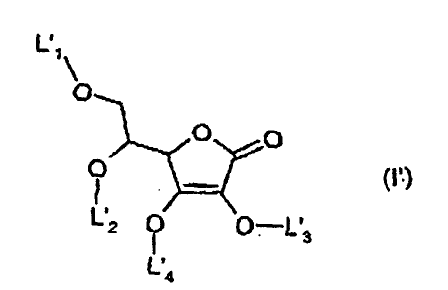 in which L'₁, L'₂, L'₃ and L'₄ represent benzyloxy groups or correspond to one of the following two formulae (b) and (b'): 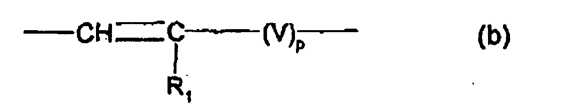 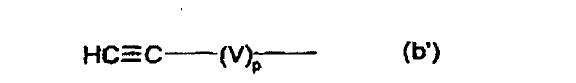 in which R₁, V and p have the same meanings as above, it being possible for the radicals L'₁ and L'₂ in addition to form together with the ascorbic acid residue a methylenedioxy ring substituted with at least one C₁₋₆ alkyl group or a phenyl group, in particular with a methyl, ethyl or phenyl group, or even with two alkyl or phenyl groups;
with the proviso that at least one of the radicals L'₁, L'₂, L'₃ and L'₄ represents the radical (b) or (b'); followed by deprotection of the compound obtained, for example by acid hydrolysis of the isopropylidenes and/or debenzylation by catalytic hydrogenation.

9. Method of preparing the silane derivatives of formula (2) by reacting a derivative of formula (I''') : 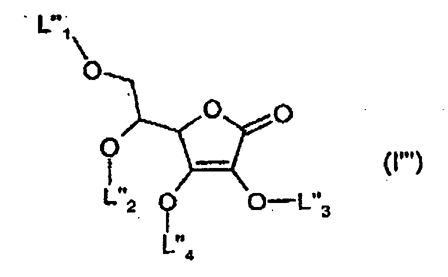 in which L''₁, L''₂, L''₃ and L''₄ represent benzyloxy groups or a hydrogen atom,
it being possible for the radicals L''₁, L''₂ in addition to form together with the ascorbic acid residue a methylenedioxy ring substituted with at least one C₁₋₆ alkyl group or a phenyl group, in particular with a methyl, ethyl or phenyl group, or even with two alkyl or phenyl groups;
with the proviso that at least one of the radicals L''₁, L''₂, L''₃ and L''₄ represents hydrogen,
with a silane derivative of the following formula (8) : 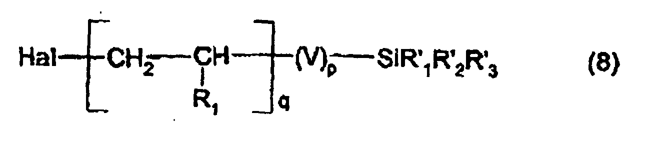 in which Hal represents a halogen and more particularly chlorine or iodine and the radicals R₁, R'₁, R'₂, R'₃, V, p and q have the same meanings as above.

10. Composition, in particular a cosmetic or pharmaceutical composition, comprising a cosmetically or pharmaceutically acceptable medium and at least one compound according to one of Claims 1 to 7.

11. Composition according to Claim 10, in which the compound is present in an amount of 0.1% to 10% by weight, preferably between 0.5% and 5% by weight, relative to the total weight of the composition.

12. Composition according to either of Claims 10 and 11, provided in the form of a composition to be applied to the skin of the body and/or of the face, and/or to the hair, eyelashes, eyebrows and/or nails.

13. Composition according to one of Claims 10 to 12, provided in the form of a composition for protecting the human epidermis or the hair against UV radiation (antisun compositions); of a cosmetic composition for the protection or treatment or care of the hair, in particular in the form of a shampoo, lotion, gel or rinse-off composition, to be applied before or after shampooing, before or after dyeing or bleaching, before, during or after permanent-waving or hair straightening, of a hair-styling or treatment lotion or gel, of a blow-drying or hair-setting lotion or gel, of a hair lacquer, of a composition for permanent-waving or hair straightening, for dyeing or bleaching the hair; of a care composition for the skin of the body and/or of the face, such as a treatment cream for the epidermis, day cream, night cream, antiwrinkle cream, moisturizing cream, cream for the hands or the feet; of a makeup composition for the eyelashes, the eyebrows, the hair, the body or the face, such as foundation, lipstick, eyeshadow, blusher, eyeliner, mascara, dyeing gel, nail varnish.

14. Use of at least one compound according to one of Claims 1 to 7, for the preparation of a pharmaceutical composition intended for treating oxidative stress and/or treating the effects of exposure to ionizing or solar radiation, and/or preventing the ageing in particular of the skin, of the hair, of the eyelashes, of the eyebrows and/or of the nails, and/or treating the effects of the use of certain medicaments which generate free radicals.
